# EUROPÄISCHE PATENTSCHRIFT

(11) **EP 1 296 642 B1**
(45) Veröffentlichungstag und Bekanntmachung des Hinweises auf die Patenterteilung: **26.10.2011**
(21) Anmeldenummer: 01960432.1
(22) Anmeldetag: 30.06.2001
(51) Int. Cl.: A61K 31/195, A61K 8/44, A61Q 17/04

(54) **VERWENDUNG VON KREATIN UND/ODER KREATINDERIVATEN IN DERMATOLOGISCHEN ZUBEREITUNGEN**
USE OF CREATINE AND/OR CREATINE DERIVATIVES IN DERMATOLOGICAL PREPARATIONS
UTILISATION DE CREATINE ET/OU DE DERIVES DE CREATINE DANS DES PREPARATIONS DERMATOLOGUES

(30) Priorität: 06.07.2000 DE 10032964
(43) Veröffentlichungstag der Anmeldung: 02.04.2003
(60) Teilanmeldung: 05025279.0
(73) Patentinhaber: Beiersdorf AG, 20245 Hamburg (DE)
(72) Erfinder: BLATT, Thomas, 22880 Wedel (DE); SCHMIDT, Melanie, 21031 Hamburg (DE); SCHREINER, Volker, 20259 Hamburg (DE); STÄB, Franz, 21379 Echem (DE)
(86) Internationale Anmeldenummer: PCT/EP2001/007510
(87) Internationale Veröffentlichungsnummer: WO 2002/002075

(56) Entgegenhaltungen:
- EP-A- 0 413 528
- EP-A- 0 565 010
- EP-A- 0 852 949
- WO-A-01/00203
- WO-A-01/03325
- WO-A-01/21208
- WO-A-95/19769
- DE-A- 19 533 330
- DE-A- 19 841 385

## Beschreibung

Die vorliegende Erfindung betrifft die Verwendung von Kreatin und/oder Kreatinderivaten in kosmetischen oder dermatologischen Zubereitungen zur Behandlung und Prophylaxe der Symptome von UV- und oder Ozon- induzierten Hautschäden, sowie gegebenenfalls zusätzlichen von entzündlichen und degenerativen Hautzuständen.

Unter kosmetischer Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).
   Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der durch Umweltnoxen wie z.B. UV-Licht, Ozon, Zigarettenrauch gestressten Haut, sowie zur Behandlung der Folgeschäden der Lichtalterung, insbesondere der unter a) bis g) aufgeführten Phänomene.

Produkte zur Pflege gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere vergleichend erwähnt werden kosmetische Zubereitungen mit einem wirksamen Schutz vor schädlichen Oxidationsprozessen in der Haut, aber auch zum Schutze kosmetischer Zubereitungen selbst bzw. zum Schutze der Bestandteile kosmetischer Zubereitungen vor schädlichen Oxidationsprozessen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, des sogenannten UVA-Bereichs, ist es wichtig, Filtersubstanzen zur Verfügung zu haben, da dessen Strahlen Reaktionen bei lichtempfindlicher Haut hervorrufen können. Es ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)), nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Um diesen Reaktionen vorzubeugen, können den dermatologischen Formulierungen zusätzliche Antioxidantien und/oder Radikalfänger einverleibt werden.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Aufgabe der Erfindung war es daher dermatologische und pharmazeutische Wirkstoffe und Zubereitungen sowie Lichtschutzformulierungen zu schaffen, die zur Prophylaxe und Behandlung lichtempfindlicher Haut, insbesondere Photodermatosen, bevorzugt PLD dienen.

Weitere Bezeichnungen für die polymorphe Lichtdermatose sind PLD, PLE, Mallorca-Akne und eine Vielzahl von weiteren Bezeichnungen, wie sie in der Literatur (z.B. A. Voelckel et al, Zentralblatt Haut- und Geschlechtskrankheiten (1989), 156, S.2), angegeben sind.

Hauptsächlich werden Antioxidantien als Schutzsubstanzen gegen den Verderb der sie enthaltenden Zubereitungen verwendet. Dennoch ist bekannt, daß auch in der menschlichen und tierischen Haut unerwünschte Oxidationsprozesse auftreten können.

Im Aufsatz "Skin Diseases Associated with Oxidative Injury" in "Oxidative Stress in Dermatology", S. 323 ff. (Marcel Decker Inc., New York, Basel, Hong Kong, Herausgeber: Jürgen Fuchs, Frankfurt, und Lester Packer, Berkeley/Californien), werden oxidative Schäden der Haut und ihre näheren Ursachen aufgeführt.

Aus EP0413528 ist bekannt, dass amphoterisch wirkende Verbindungen wie Kreatin in kosmetischen Zubereitungen zur Behandlung von Hautschäden sowie von entzündlichen und degenerativen Hautzuständen zum Einsatz kommen.

Auch aus dem Grunde, solchen Reaktionen vorzubeugen, können kosmetischen oder dermatologischen Formulierungen zusätzlich Antioxidantien und/oder Radikalfänger einverleibt werden.

Zwar sind einige Antioxidantien und Radikalfänger bekannt. So ist bereits in den US-Patentschriften 4,144,325 und 4,248,861 sowie aus zahlreichen anderen Dokumenten vorgeschlagen worden, Vitamin E, eine Substanz mit bekannter antioxidative Wirkung in Lichtschutzformulierungen einzusetzen, dennoch bleibt auch hier die erzielte Wirkung weit hinter der erhofften zurück.

Aufgabe der vorliegenden Erfindung war es somit, Wege zu finden, die die Nachteile des Standes der Technik vermeiden. Insbesondere soll die Wirkung der Behebung der durch Umweltnoxen verursachten Schäden und die Prophylaxe dauerhaft, nachhaltig und ohne das Risiko von Nebenwirkungen sein.

Diesen Übelständen abzuhelfen, war Aufgabe der vorliegenden Erfindung.

Es hat sich überraschenderweise herausgestellt, daß die Verwendung von Kreatin und/oder Kreatinderivaten in kosmetischen oder dermatologischen Zubereitungen zur Behandlung und Prophylaxe der Symptome von UV- und oder Ozon- induzierten Hautschäden sowie gegebenenfalls zusätzlich von entzündlichen und degenerativen Hautzuständen den Nachteilen das Standes der Technik abhilft.

Kreatin (von grch.: τo κρεασ = "das Fleisch") zeichnet sich durch folgende Struktur aus:

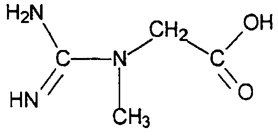

Es findet sich im Muskelsaft der Wirbeltiere zu 0,05-0,4%, in geringen Mengen auch im Gehirn und Blut. Als Monohydrat stellt es ein farbloses, kristallines Pulver dar. In wäßriger Lösung wird Kreatinin gebildet.. Im Organismus entsteht es durch Transamidinierung von L-Arginin auf Glycin zu Guanidinoessigsäure und deren anschließende Methylierung mittels S-Adenosylmethionin (durch Guanidinoacetat-Methyltransferase). Man hält Kreatin für einen appetitfördemden Bestandteil von Rindfleisch und Fleischextrakt. Kreatinzusatz zur Nahrung verstärkt die körperliche Leistungsfähigkeit.

Bevorzugt enthalten dermatologische Zubereitungen gemäß der Erfindung 0,001 - 10 Gew.-%, besonders bevorzugt 0,01 - 1 Gew.-%, an Kreatin und/oder Kreatinderivaten, bezogen auf die Gesamtzusammensetzung der Zubereitungen.

Bevorzugtes Derivat ist das Kreatinphosphat, welches folgende Struktur aufweist: 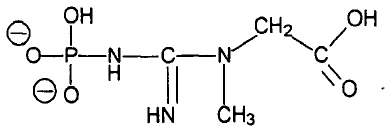 und welches im frischen Muskel verbreitet ist und dort als energiespeichemdes Phosphat (Phosphagen) eine wichtige Rolle spielt. Im arbeitenden Muskel gibt Kreatinphosphat mit Adenosin-5'-diphosphat unter dem Einfluß des Enzyms Kreatin-Kinase Adenosin-5'-triphosphat (ATP) und Kreatin; im ruhenden Muskel läuft die umgekehrte Reaktion ab.

Aber auch Kreatinsulfat, Kreatinacetat, Kreatinascorbat und die an der Carboxylgruppe mit mono- oder polyfunktionalen Alkoholen veresterten Derivate führen zu vorteilhaften Ausführungsformen der Erfindung.

Bei Anwendung des erfindungsgemäß verwendeten Wirkstoffes bzw. kosmetischer oder topischer dermatologischer Zubereitungen mit einem wirksamen Gehalt an erfindungsgemäß verwendetem Wirkstoff ist in überraschender Weise eine wirksame Behandlung, aber auch eine Prophylaxe
- von defizitären, sensitiven oder hypoaktiven Hautzuständen oder defizitären, sensitiven oder hypoaktiven Zustände von Hautanhangsgebilden
- von bestimmten degenerativen Erscheinungen der Haut (z.B. Hauterschlaffung, Altersflecken, Teleangiektasien sowie Schwund der epidermalen und dermalen Zellschichten, der Bestandteile des Bindegewebes, der Retezapfen und Kapillargefässe der Haut) und/oder der Hautanhangsgebilde,
- von umweltbedingten (Rauchen, Smog, reaktive Sauerstoffspecies, freie Radikale) und insbesondere lichtbedingten negativen Veränderungen der Haut und der Hautanhangsgebilde.
- von lichtbedingten Hautschäden
- von Pigmentierungsstörungen,
- von Juckreiz,
- von Hornschichtbarrierestörungen,
- von Haarausfall und für verbessertes Haarwachstum
- von entzündlichen Hautzuständen sowie atopischem Ekzem, seborrhoischem Ekzem, polymorpher Lichtdermatose, Psoriasis, Vitiligo
   möglich. Der erfindungsgemäße Wirkstoff bzw. kosmetische oder topische dermatologische Zubereitungen mit einem wirksamen Gehalt an erfindungsgemäßem Wirkstoff dient aber auch in überraschender Weise
- zur Beruhigung von empfindlicher oder gereizter Haut
- zur Stimulation der Kollagen-, Hyaluronsäure-, Elastinsynthese
- zur Stimulation der intrazellulären DNA-Synthese, insbesondere bei defizitären oder hypoaktiven Hautzuständen.
- zur Steigerung der Zellemeuerung und Regeneration der Haut
- zur Steigerung der hauteigenen Schutz- und Reparaturmechanismen (beispielsweise für dysfunktionelle Enzyme, DNA, Lipide, Proteine)
- zur Vor- und Nachbehandlung bei topischer Anwendung von Laser- und Abschleifbehandlungen, die z. B. der Reduzierung von Hautfalten und Narben dienen, um den resultierenden Hautreizungen entgegenzuwirken und die Regenerationsprozesse in der verletzten Haut zu fördern.

Es ist erfindungsgemäß insbesondere äußerst vorteilhaft, den erfindungsgemäß verwendeten topischten dermatologischen Zubereitungen mit einem wirksamen Gehalt an erfindungsgemäß verwendetem Wirkstoff zur dermatologischen Behandlung oder Prophylaxe unerwünschter Hautzustände zu verwenden.

Erfindungsgemäß können Zubereitungen, welche die erfindungsgemäßen Wirkstoffkombinationen enthalten, übliche Antioxidantien eingesetzt werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Alanindiessigsäure, Flavonoide, Polyphenole, Catechine, Vitamin C und Derivate (z.B. Ascorbylpalmitat, Mg-Ascorbylphosphat, Ascorbylacetat), Tocopherole und Derivate (z.B. Vitamin-E-acetat), sowie Koniferylbenzoat des Benzoäharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Die Prophylaxe bzw. dermatologische Behandlung mit dem erfindungsgemäß verwendeten Wirkstoff bzw. mit den topischen dermatologischen Zubereitungen mit einem wirksamen Gehalt an erfindungsgemäß verwendetem Wirkstoff erfolgt in der üblichen Weise, und zwar dergestalt, daß der erfindungsgemäß verwendete Wirkstoff bzw. die topischen dermatologischen Zubereitungen mit einem wirksamen Gehalt an erfindungsgemäß verwendetem Wirkstoff auf die betroffenen Hautstellen aufgetragen wird.

Vorteilhaft kann der erfindungsgemäß verwendete Wirkstoff eingearbeitet werden in übliche dermatologische Zubereitungen, welche in verschiedenen Formen vorliegen können. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W) oder Öl-in-Wasser-in-Öl (O/W/O), eine Hydrodispersion oder Lipodispersion, ein Gel, einen festen Stift oder auch ein Aerosol darstellen.

Erfindungsgemäße Emulsionen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, den erfindungsgemäß verwendeten Wirkstoff in wäßrige Systeme bzw. Tensidzubereitungen zur Reinigung der Haut und der Haare einzufügen.

Es ist dem Fachmanne natürlich bekannt, daß anspruchsvolle kosmetische Zusammensetzungen zumeist nicht ohne die üblichen Hilfs- und Zusatzstoffe denkbar sind. Darunter zählen beispielsweise Konsistenzgeber, Füllstoffe, Parfum, Farbstoffe, Emulgatoren, zusätzliche Wirkstoffe wie Vitamine oder Proteine, Lichtschutzmittel, Stabilisatoren, Insektenrepellentien, Alkohol, Wasser, Salze, antimikrobiell, proteolytisch oder keratolytisch wirksame Substanzen usw.

Mutatis mutandis gelten entsprechende Anforderungen an die Formulierung medizinischer Zubereitungen.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Entsprechend können topische dermatologische Zusammensetzungen im Sinne der vorliegenden Erfindung, je nach ihrem Aufbau, beispielsweise verwendet werden als Hautschutzcrème, Reinigungsmilch, Sonnenschutzlotion, Nährcreme, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind gegebenenfalls auch dermatologischen Zubereitungen, die in der Form eines Sonnenschutzmittels vorliegen. Vorzugsweise enthalten diese neben dem erfindungsgemäß verwendeten Wirkstoff zusätzlich mindestens eine UVA-Filtersubstanz und/oder mindestens eine UVB-Filtersubstanz und/oder mindestens ein anorganisches Pigment.

Es ist aber auch vorteilhaft im Sinne der vorliegenden Erfindung, solche dermatologische Zubereitungen zu erstellen, deren hauptsächlicher Zweck nicht der Schutz vor Sonnenlicht ist, die aber dennoch einen Gehalt an UV-Schutzsubstanzen enthalten. So werden beispielsweise in Tagescrèmes gewöhnlich UV-A- bzw. UV-B-Filtersubstanzen eingearbeitet.

Erfindungsgemäße Zubereitungen enthalten Substanzen, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen.

Die UVB-Filter können öllöslich oder wasserlöslich sein. Als öllösliche Substanzen sind z.B. zu nennen:
- 3-Benzylidencampher und dessen Derivate, z.B. 3-(4-Methylbenzyliden)campher,
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure-(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Als wasserlösliche Substanzen sind vorteilhaft:
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die erfindungsgemäß Verwendung finden können, soll selbstverständlich nicht limitierend sein.

Gegenstand der Erfindung ist die Kombination eines erfindungsgemäßen UVA-Filters mit einem UVB-Filter bzw. eine erfindungsgemäße dermatologische Zubereitung, welche auch einen UVB-Filter enthält.

Es kann auch von Vorteil sein, in erfindungsgemäßen Zubereitungen UVA-Filter einzusetzen, die üblicherweise in kosmetischen und/oder dermatologischen Zubereitungen enthalten sind. Bei solchen Filtersubstanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion. Auch Zubereitungen, die diese Kombinationen enthalten, sind Gegenstand der Erfindung. Es können die gleichen Mengen an UVA-Filtersubstanzen verwendet werden, welche für UVB-Filtersubstanzen genannt wurden.

Kosmetische und/oder dermatologische Zubereitungen im Sinne der vorliegenden Erfindung können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid. Es können die für die vorstehenden Kombinationen genannten Mengen verwendet werden.

Die erfindungsgemäßen dermatologischen Zubereitungen können kosmetische Wirk-, Hilfs- und/oder Zusatzstoffe enthalten, wie sie üblicherweise in solchen Zubereitungen verwendet werden, z.B. Antioxidationsmittel, Konservierungsmittel, Bakterizide, Parfüme, Substanzen zum Verhindern des Schäumen, Farbstoffe, Pigmente, die färbende Wirkung haben, Verdickungsmittel, oberflächenaktive Substanzen, Emulgatoren, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Sofern die dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbeältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Zubereitungen im Sinne der vorliegenden Erfindung können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel, z.B. Gummiarabikum, Xanthangummi, Natriumalginat, CelluloseDerivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die nachfolgenden Beispiele sollen die vorliegende Erfindung verdeutlichen.

### Beispiel 1

### W/O Creme

| | Gew.-% |
|---|---|
| Paraffinöl (DAB 9) | 10,00 |
| Petrolatum | 4,00 |
| Wollwachsalkohol | 1,00 |
| PEG-7-Hydriertes Rizinusöl | 3,00 |
| Aluminiumstearat | 0,40 |
| Glycerin | 2,00 |
| Konservierungsmittel, Farbstoffe, Parfüm | q.s. |
| Phosphokreatin | 0,20 |
| Wasser | ad 100,00 |

### Beispiel 2

### W/O Lotion

| | Gew.-% |
|---|---|
| Paraffinöl (DAB 9) | 20,00 |
| Petrolatum | 4,00 |
| Glucosesesquiisostearat | 2,00 |
| Aluminiumstearat | 0,40 |
| Phytoen | 1,00 |
| Glycerin | 5,00 |
| Konservierungsmittel, Farbstoffe, Parfüm | q.s. |
| Kreatin | 2,50 |
| Wasser | ad 100,00 |

### Beispiel 3

### O/W Lotion

| | Gew.-% |
|---|---|
| Paraffinöl (DAB 9) | 8,00 |
| Isopropylpalmitat | 3,00 |
| Petrolatum | 4,00, |
| Cetylstearylalkohol | 2,00 |
| PEG 40 Rizinusöl | 0,50 |
| Natriumcetylstearylsulfat | 0,50 |
| Natrium Carbomer | 0,40 |
| Glycerin | 3,00 |
| α-Glucosylrutin | 0,20 |
| Octylmethoxycinnamat | 5,00 |
| Butylmethoxydibenzoylmethan | 1,00 |
| Konservierungsmittel, Farbstoffe, Parfüm | q.s. |
| Kreatinacetat | 0,02 |
| Wasser | ad 100,00 |

### Beispiel 4

### O/W Creme

| | Gew.-% |
|---|---|
| Paraffinöl (DAB 9) | 7,00 |
| Avocadoöl | 4,00 |
| Glycerylmonostearat | 2,00 |
| Natriumlactat | 3,00 |
| Glycerin | 3,00 |
| Konservierungsmittel, Farbstoffe, Parfüm | q.s. |
| Phosphokreatin | 0.4 |
| Wasser | ad 100,00 |

### Beispiel 5

### Liposomenhaltiges Gel

| | Gew.-% |
|---|---|
| Lecithin | 6,00 |
| Schibutter | 3,00 |
| Taurin | 0,20 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Konservierungsmittel, Farbstoffe, Parfüm | q.s. |
| Kreatin | 1,20 |
| Wasser | ad 100,00 |

### Beispiel 6

### Sonnenschutzemulsion

| | Gew.-% |
|---|---|
| Cyclomethicon | 2,00 |
| Cetyldimethicon Copolyol | 0,20 |
| PEG 22-Dodecyl Copolymer | 3,00 |
| Paraffinöl (DAB 9) | 2,00 |
| Caprylsäure-/Caprinsäure Triglycerid | 5,80 |
| Octylmethoxycinnamat | 5,80 |
| Butyl-methoxy-dibenzoylmethan | 4,00 |
| Phospokreatin | 0,50 |
| D-Biotin | 0,50 |
| ZnSO₄ | 0,70 |
| Na₄EDTA | 0,30 |
| Konservierungsmittel, Farbstoffe, Parfüm | q.s. |
| Wasser | ad 100,00 |

### Beispiel 7

### Sonnenschutzemulsion

| | Gew.-% |
|---|---|
| Cyclomethicon | 2,00 |
| Cetylstearylalkohol + PEG 40-hydriertes Rizinusöl + Natrium Cetylstearylsulfat | 2,50 |
| Glyceryllanolat | 1,00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,50 |
| Kreatin | 0,20 |
| a-Tocopherylacetat | 1,00 |
| Na₃HEDTA | 1,50 |
| Konservierungsmittel, Farbstoffe, Parfüm | q.s. |
| Wasser | ad 100,00 |

### Beispiel 8

### Sonnenschutzemulsion

| | Gew.-% |
|---|---|
| Cyclomethicone | 2,00 |
| Cetylstearylalkohol +PEG 40-hydriertes Rizinusöl +Natrium Cetylstearylsulfat | 2,50 |
| Glyceryllanolat | 1.00 |
| Caprylsäure-/Caprinsäure Triglycerid | 0,10 |
| Laurylmethicon Copolyol | 2,00 |
| Octylstearat | 3,00 |
| Rizinusöl | 4,00 |
| Glycerin | 3,00 |
| Acrylamid/Natriumacrylat Copolymer | 0,30 |
| Hydroxypropylmethylcellulose | 0,30 |
| Octylmethoxycinnamat | 5,00 |
| Butyl-methoxy-dibenzoylmethan | 0,75 |
| Na₃HEDTA | 1,50 |
| Konservierungsmittel, Farbstoffe, Parfüm | q.s. |
| O-Acetyl-L-Camitin | 3,00 |
| Wasser | ad 100,00 |

### Beispiel 9

### Sprayformulierung

| | Gew.-% |
|---|---|
| Ubiquinon 10 | 0,10 |
| Kreatin | 0,80 |
| Ethanol | 28,20 |
| Konservierungsmittel, Farbstoffe, Parfüm | q.s. |
| Propan/Butan 25/75 | ad 100,00 |

### Beispiel 10

### O/W Creme

| | Gew.-% |
|---|---|
| Paraffinöl (DAB 9) | 7,00 |
| Soyaöl | 4,00 |
| Glycerylmonostearat | 2,00 |
| Natriumlactat | 3,00 |
| Glycerin | 8,00 |
| Konservierungsmittel, Farbstoffe, Parfüm | q.s. |
| Phosphokreatin | 0.08 |
| Wasser | ad 100,00 |

## Patentansprüche

1. Verwendung von Kreatin und/oder Kreatinderivaten zur Herstellung dermatologischer Zubereitungen zur Behandlung und Prophylaxe der Symptome von UV- und/oder Ozon- induzierten Hautschäden, wobei die Zubereitungen zusätzlich einen oder mehrere UVA- und/oder UVB-Filtersubstanzen enthalten.

2. Verwendung nach Anspruch 1, **dadurch gekennzeichnet, daß** der oder die UVB-Filter gewählt wird oder werden aus der Gruppe
- 3-Benzylidencampher und dessen Derivate, z.B. 3-(4-Methylbenzyliden)campher,
- 4-Aminobenzoesäure-Derivate, vorzugsweise 4-(Dimethylamino)-benzoësäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester,
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.
- 2-Phenylbenzimidazol-5-sulfonsäure und deren Salze, z.B. Natrium-, Kalium- oder Triethanolammonium-Salze,
- Sulfonsäure-Derivate von Benzophenonen, vorzugsweise 2-Hydroxy-4-methoxybenzophenon-5-sulfonsäure und ihre Salze;
- Suifonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

3. Verwendung nach einem der vorstehenden Ansprüche, **dadurch gekennzeichnet, daß** der oder die UVA-Fifter gewählt wird oder werden aus der Gruppe der Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)prapan-1,3-dion.

4. Verwendung nach einem der vorstehenden Ansprüche, wobei das Kreatinderivat gewählt wird aus der Gruppe Kreatinphosphat, Kreatinsulfat, Kreatinascorbat, Kreatinacetat gewählt wird.

5. Verwendung nach einem der vorstehenden Ansprüche, wobei die dermatologischen Zubereitungen 0,001 - 0,49 Gew.-% bzw. 10,1 - 30 Gew.% an Kreatin und/oder Kreatinderivaten enthalten, bezogen auf das Gesamtgewicht der Zubereitungen.

6. Verwendung nach einem der vorstehenden Ansprüche, wobei die dermatologischen Zubereitungen sich durch einen zusätzlichen Gehalt (0,0001 - 30 Gew. %) an einem oder mehreren der folgenden natürlichen Wirkstoffen und deren Derivate auszeichnen (alpha-Liponsäure, Phytoen, D-Biotin, Coenzym Q10, alpha Glucosylrutin, Carnitin, Carnosin, Isoflavon).

7. Verwendung nach einem der vorstehenden Ansprüche zur Behandlung und Prophylaxe der Symptome von UV- und oder Ozon- induzierten Hautschäden sowie von entzündlichen und degenerativen Hautzuständen.

## Claims

1. Use of creatine and/or creatine derivatives for producing dermatological preparations for the treatment and prophylaxis of the symptoms of UV- and/or ozone-induced skin damage, where the preparations additionally comprise one or more UVA and/or UVB filter substances.

2. Use according to Claim 1, **characterized in that** the UVB filter or filters is or are selected from the group
- 3-benzylidenecamphor and derivatives thereof, e.g. 3-(4-methylbenzylidene)camphor,
- 4-aminobenzoic acid derivatives, preferably 2-ethylhexyl 4-(dimethylamino)benzoate, amyl 4-(dimethylamino)benzoate;
- esters of cinnamic acid, preferably 2-ethylhexyl 4-methoxycinnamate, isopentyl 4-methoxycinnamate;
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydxoxy-4-methoxy-4'-methylbenzophenone, 2,2'-dihydroxy-4-methoxybenzophenone;
- esters of benzalmalonic acid, preferably di(2-ethylhexyl) 4-methoxybenzalmalonate;
- 2,4,6-trianilino(p-carbo-2'-ethyl-1'-hexyloxy)-1,3,5-triazine;
- 2-phenylbenzimidazole-5-sulphonic acid and salts thereof, e.g. sodium, potassium or triethanolammonium salts,
- sulphonic acid derivatives of benzophenones, preferably 2-hydroxy-4-methoxybenzophenone-5-sulphonic acid and its salts;
- sulphonic acid derivatives of 3-benzylidenecamphor, such as e.g. 4-(2-oxo-3-bornylidenemethyl)benzenesulphonic acid, 2-methyl-5-(2-oxo-3-bornylidenemethyl)sulphonic acid and its salts.

3. Use according to one of the preceding claims, **characterized in that** the UVA filter or filters is or are selected from the group of the derivatives of dibenzoylmethane, in particular 1-(4'-tert-butylphenyl)-3-(4'-methoxyphenyl)propane-1,3-dione and 1-phenyl-3-(4'-isopropylphenyl)propane-1, 3-dione.

4. Use according to one of the preceding claims, where the creatine derivative is selected from the group creatine phosphate, creatine sulphate, creatine ascorbate, creatine acetate.

5. Use according to one of the preceding claims, where the dermatological preparations comprise 0.001-0.49% by weight or 10.1-30% by weight of creatine and/or creatine derivatives, based on the total weight of the preparations.

6. Use according to one of the preceding claims, where the dermatological preparations are distinguished by an additional content (0.0001-30% by weight) of one or more of the following natural active ingredients and derivatives thereof (alpha-lipoic acid, phytoene, D-biotin, coenzyme Q10, alpha-glucosylrutin, carnitine, carnosine, isoflavone).

7. Use according to one the preceding claims for the treatment and prophylaxis of the symptoms of UV- and/or ozone-induced skin damage, and also of inflammatory and degenerative skin conditions.

## Revendications

1. Utilisation de créatine et/ou de dérivés de créatine pour la fabrication de préparations dermatologiques destinées au traitement et à la prophylaxie des symptômes de lésions cutanées induites par les UV et/ou l'ozone, les préparations contenant en outre une ou plusieurs substances filtrant les UVA et/ou les UVB.

2. Utilisation selon la revendication 1, **caractérisée en ce que** le ou les filtres UVB est ou sont choisis dans le groupe constitué par
- le 3-benzylidènecamphre et ses dérivés, par exemple le 3-(4-méthylbenzylidène)camphre,
- des dérivés de l'acide 4-aminobenzoïque, de préférence le 4-(diméthylamino)benzoate de 2-éthylhexyle, le 4-(diméthylamino)benzoate d'amyle ;
- des esters de l'acide cinnamique, de préférence le 4-méthoxycinnamate de 2-éthylhexyle, le 4-méthoxycinnamate d'isopentyle ;
- des esters de l'acide salicylique, de préférence le salicylate de 2-éthylhexyle, le salicylate de 4-isopropylbenzyle, le salicylate d'homomenthyle ;
- des dérivés de la benzophénone, de préférence la 2-hydroxy-4-méthoxybenzophénone, la 2-hydroxy-4-méthoxy-4'-méthylbenzophénone, la 2,2'-dihydroxy-4-méthoxybenzophénone ;
- des esters de l'acide benzalmalonique, de préférence le 4-méthoxybenzalmalonate de di(2-éthylhexyle) ;
- la 2,4,6-trianilino-(p-carbo-2'-éthyl-l'-hexyloxy)-1,3,5-triazine,
- l'acide 2-phénylbenzimidazole-5-sulfonique et ses sels, par exemple les sels de sodium, potassium ou triéthanolammonium,
- des dérivés sulfonés de la benzophénone, de préférence l'acide 2-hydroxy-4-méthoxybenzophénone-5-sulfonique et ses sels ;
- des dérivés sulfonés du 3-benzylidènecamphre, comme par exemple l'acide 4-(2-oxo-3-bornylidène-méthyl)-benzènesulfonique, l'acide 2-méthyl-5-(2-oxo-3-bornylidène-méthyl)sulfonique et leurs sels.

3. Utilisation selon l'une quelconque des revendications précédentes, **caractérisée en ce que** le ou les filtres UVA est ou sont choisis dans le groupe des dérivés du dibenzoylméthane, en particulier la 1-(4'-tert-butylphényl)-3-(4'-méthoxyphényl)propane-1,3-dione et le 1-phényl-3-(4'-isopropylphényl)propane-1,3-dione.

4. Utilisation selon l'une quelconque des revendications précédentes, dans laquelle le dérivé de créatine est choisi dans le groupe constitué par le phosphate de créatine, le sulfate de créatine, l'ascorbate de créatine, l'acétate de créatine.

5. Utilisation selon l'une quelconque des revendications précédentes, dans laquelle les préparations dermatologiques contiennent 0,001 - 0,49 % en poids ou 10, 1 - 30 % en poids de créatine et/ou de dérivés de créatine, par rapport au poids total des préparations.

6. Utilisation selon l'une quelconque des revendications précédentes, dans laquelle les préparations dermatologiques se distinguent par une teneur supplémentaire (0,0001 - 30 % en poids) en une ou plusieurs des substances actives naturelles suivantes et leurs dérivés (acide alpha-lipoïque, phytoène, D-biotine, coenzyme Q10, alpha-glucosylrutine, carnitine, carnosine, isoflavone).

7. Utilisation selon l'une quelconque des revendications précédentes, pour le traitement et la prophylaxie des symptômes de lésions cutanées induites par les UV et/ou l'ozone ainsi que d'états inflammatoires et dégénératifs de la peau.
